# EUROPEAN PATENT SPECIFICATION

(11) **EP 2 742 840 B1**
(45) Date of publication and mention of the grant of the patent: **19.04.2017**
(21) Application number: 13196648.3
(22) Date of filing: 11.12.2013
(51) Int. Cl.: A61L 2/18, A61L 9/03

(54) **Hand dryer**
Handtrockner
Sèche-mains

(30) Priority: 11.12.2012 ES 201231926
(43) Date of publication of application: 18.06.2014
(73) Proprietor: Angarita Duran, Liz Johanna, 03540 Alicante (ES)
(72) Inventor: Angarita Duran, Liz Johanna, 03540 Alicante (ES)
(74) Representative: Del Valle Valiente, Sonia

(56) References cited:
- DE-U1-202012 006 801
- GB-A- 2 194 442
- GB-A- 2 413 283

## Description

### OBJECT OF THE INVENTION

The present invention refers to a hand dryer which, in addition to drying the hands by means of a warm air exit, also propels a sprayed germicidal or bactericidal liquid, or any other personal hygiene product. Additionally, the dryer incorporates an air freshener for emitting scents as a part of the housing thereof.

It is thus an object of the invention to provide a hand dryer which also carries out hygienic functions and which also allows for establishing an evaporation air freshener forming part of the dryer assembly.

### BACKGROUND OF THE INVENTION

It is known that hand dryers that function by propelling warm air are based on actuating a turbine propelling air through an electrical resistor, whereby the air is heated and exits through a port or nozzle under which the hands to be dried are placed.

These hand dryers do not perform any function other than drying the hands by means of warm air, not providing any hygienic function of the hands to be dried or of course emitting a scent by evaporation, as the dryer which is the object of the present invention.

Document GB 2413283 discloses a washroom scent delivery apparatus for use in conjunction with a hot air dryer where part of the air stream used for drying the hands of a user is diverted towards a scent outlet.

Document GB 2194442 discloses a heat fumigation apparatus disclosing a liquid chemical drawn up by means of a wick whose upper portion is heated by a heater.

Document DE 202012006801 discloses a hand dryer where an air stream causes the suction of a bacterial liquid stored in a container.

### DESCRIPTION OF THE INVENTION

The hand dryer is formed by a housing having two parts which are completely independent and isolated therebetween, one corresponding to the elements participating in the air propulsion, heating thereof and suitable control and actuation means, while the other part which is isolated and independent from the first part includes a container of a germicidal or bactericidal liquid which allows for the introduction of said liquid towards the warm air exit zone by means of a duct having solenoid valve suitable controlled by means of an electronic circuit, thereby causing the emission of liquid molecules, and where the liquid in the germicidal container is sucked by the Venturi effect that appears in the warm air exit zone.

Therefore, in the first place the hand dryer not only carries out a drying function by means of warm air, but it also sprays liquid molecules of a germicidal bactericidal product contained in a container which is isolated and independent with respect to the plurality of components participating in the hand dryer itself.

Additionally, the part of the housing which is independent from the hand dryer, where the germicidal bactericidal container is located, includes an air freshener formed by a deposit of scent mounted on a support, having an outside air access and a wick absorbing the liquid scent from the deposit, and by means of a heating element controlled by a regulation potentiometer, the aromatic liquid is evaporated and exits towards the outside of the air freshener through a grille located in the upper part, therefore the dryer, in addition to the two functions disclosed above, also fulfils an air freshener function by means of the evaporation of an aromatic liquid.

In an alternative embodiment, the duct communicating the germicidal container to the hand dryer exit zone may end directly in said zone or else in a narrow internal perimeter area having holes for obtaining a more homogeneous air-liquid mix.

### DESCRIPTION OF THE DRAWINGS

In order to complement the description below and with the object to aid in a better understanding of the features of the invention, according to an example of a preferred embodiment thereof, a set of drawings being an integral part of said description is attached where, by way of illustration and not of limitation, the following is represented:
Figure 1.- Shows a lateral view of the hand dryer which is the object of the invention with the part showing the separation of the dryer itself with respect to the zone where the air freshener is established.
Figure 2.- Shows an schematic view of the inside corresponding to the hand dryer and air freshener assembly represented in the above figure, where all the components participating in said assembly are visible.
Figure 3.- Shows an schematic view of an alternative embodiment where the duct coming from the germicidal or bactericidal liquid container ends in a narrow inside perimeter zone of the warm air exit port corresponding to the hand dryer itself.

### PREFERRED EMBODIMENT OF THE INVENTION

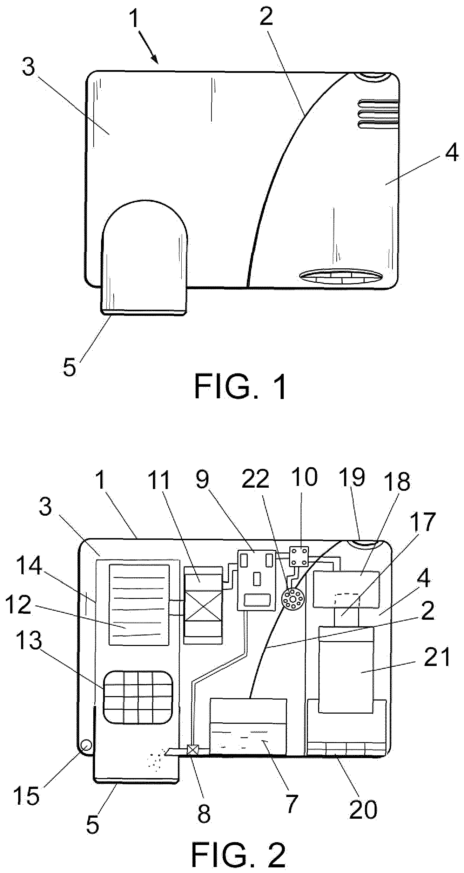

As shown in the above figures, and specifically in connection with figures 1 and 2, the hand dryer is formed by a housing (1) having a wall (2) for separating and isolating zone (3) corresponding to the hand dryer itself from zone (4) corresponding to an evaporation air freshener.
As to the hand dryer itself, it includes a port (5) for the exit of warm air, close to which a duct (6) coming from a germicidal or bactericidal liquid container (7) ends, said duct (6) having a closure solenoid valve (8) whose opening time is controllable by means of an electronic circuit (9) having its respective power supply (10), such that through said electronic circuit (9) a motor (11) is activated, said motor in turn making a turbine (12) turn for propelling air towards the exit port (5), said air having previously passed through a resistor (13) which heats the air propelled by the turbine (12), which is housed inside an internal housing (14), as shown in figure 2.
This way, air propelled by the turbine (12), after passing through the resistor (13), is heated up and exits through port (5), drying hands placed under said exit port (5), while the low pressure created in the exit zone causes the liquid from the duct (6) coming from the container (7) to be sucked by the Venturi effect, thereby producing liquid molecules which exit through the port (5), thus drying the hands and at the same time carrying out a hygienic cleaning thereof.
As in other devices of this type, an optical sensor (15) which activates when the hands are placed under the exit port (5) is included.
The air freshener (4) is placed at the other side of the separation and isolation wall (2), and it is formed by a deposit (16) which contains the freshening product which is sucked by means of a wick (17) in order to be evaporated by means of a heating element (18) and subsequently to cause the scent to exit through an upper grille (19), this assembly also having an air input (20) and a support (21) for the scent deposit (16).

A potentiometer or switch having several positions (22) allows for regulating the intensity through the resistors corresponding to the heating element (18), and therefore for regulating the evaporation capacity of the air freshener itself.

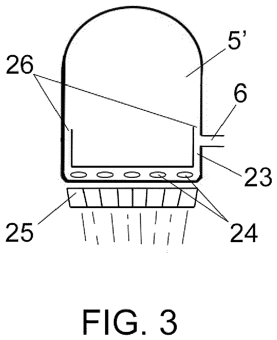

Figure 3 shows an alternative embodiment for the positioning or the way the duct (6) coming from the germicidal or bactericidal liquid deposit (7) ends at the exit zone (5') of the air freshener, such that in the case of the alternative embodiment shown in figure 3 said duct (6) ends in a narrow zone (23) through which pressurized air produces the exit of liquid molecules through the holes (24) established in the lower part or in an exit nozzle (25), as shown schematically in figure 3, where the arrows (26) indicate the pressurized air input towards the narrow (23) zones where the duct (6) from the germicidal bactericidal container (7) ends.

## Claims

1. Hand dryer which includes a turbine (12) which is actuatable by a motor (11) for propelling air which is heated by means of a resistor placed between the turbine itself and an exit port under which the hands to be dried are placed, whereby the hand dryer comprises a housing (1) in which a scent evaporation air freshener (4) is placed; where a duct coming from a germicidal bactericidal liquid container (7) ends in a warm air exit zone, such that the germicidal bactericidal liquid accesses the exit zone; where in the zone where the evaporation air freshener is established, the germicidal bactericidal liquid container is also located, said air freshener including a scent deposit which, by means of a wick (17), exits outside through an upper grille (19); (20),
**characterised in that**
the housing (1) is with a separation wall (2) which separates the elements participating in the hand dryer and with an isolated and independent zone; the scent deposit reaches a heating element (18) for evaporating the scent; there is a lower outside air entrance grille; the germicidal bactericidal liquid accesses the exit zone by means of the Venturi effect caused by the reduction of pressure, the duct being sucked; the reduction in pressure of the warm air causes spraying of the germicidal bactericidal liquid towards the exit port; and the germicidal bactericidal liquid duct includes a solenoid valve (8) whose closure is controlled by means of an electronic circuit (9) controlling the elements participating in the dryer.

2. Hand dryer according to claim 1, **characterized in that** the exit duct for the germicidal bactericidal liquid in the container has access to the dryer exit zone through a narrow zone having a pressurized air input, the air exiting outside through holes or through a lower nozzle, in order to achieve the spraying of the germicidal bactericidal liquid and its exit towards the hands for performing the hygienic cleaning function.

## Patentansprüche

1. Handtrockner, der eine Turbine (12) aufweist, die mit einem Motor (11) zum Vorwärtstreiben von Luft betätigbar ist, die mittels eines Widerstands erwärmt wird, der zwischen der Turbine selbst und einer Ausströmöffnung platziert ist, unter der die zu trocknenden Hände platziert werden, wobei der Handtrockner ein Gehäuse (1) umfasst, in dem ein Duftstoff-Verdunstungslufterfrischer (4) platziert ist; wo ein von einem Behälter für eine keimtötende, bakterientötende Flüssigkeit (7) kommender Kanal in einem Warmluftaustrittsbereich endet, sodass die keimtötende, bakterientötende Flüssigkeit in den Austrittsbereich gelangt;
wobei sich in dem Bereich, in dem der Verdunstungslufterfrischer untergebracht ist, auch der Behälter für eine keimtötende, bakterientötende Flüssigkeit befindet, wobei der Lufterfrischer einen Duftstoffvorrat aufweist, der über einen Docht (17) durch ein oberes Gitter (19); (20) nach außen strömt;
**dadurch gekennzeichnet, dass**
das Gehäuse (1) eine Trennwand (2) aufweist, die die den Handtrockner bildenden Elemente abtrennt, sowie einen isolierten und unabhängigen Bereich; der Duftstoff-Vorrat ein Heizelement (18) zum Verdunsten des Duftstoffs erreicht; ein unteres Außenluft-Einströmgitter vorhanden ist; die keimtötende, bakterientötende Flüssigkeit den Austrittsbereich über den Venturi-Effekt erreicht, der durch die Druckminderung bewirkt wird, wobei aus dem Kanal gesaugt wird; die Minderung des Drucks der warmen Luft ein Versprühen der keimtötenden, bakterientötenden Flüssigkeit in Richtung der Ausströmöffnung bewirkt; und der Kanal für die keimtötende, bakterientötende Flüssigkeit ein Magnetventil (8) aufweist, dessen Schließen mittels einer elektronischen Schaltung (9) gesteuert wird, die die den Trockner bildenden Elemente steuert.

2. Handtrockner nach Anspruch 1, **dadurch gekennzeichnet, dass** der Ausströmkanal für die keimtötende, bakterientötende Flüssigkeit in dem Behälter über einen engen Bereich mit einem Drucklufteinlass zum Trocknerausströmbereich führt, wobei die Luft durch Löcher oder durch eine untere Düse nach außen strömt, damit das Versprühen der keimtötenden, bakterientötenden Flüssigkeit und ihr Ausströmen in Richtung der Hände bewirkt wird, damit die hygienische Reinigungsfunktion ausgeführt wird.

## Revendications

1. Sèche-mains qui comporte une turbine (12) qui peut être actionnée par un moteur (11) pour l'impulsion d'air qui est chauffé au moyen d'une résistance placée entre la turbine elle-même et un orifice de sortie sous lequel les mains à sécher sont placées, le sèche-mains comprenant un boîtier (1) dans lequel est placé un purificateur d'air (4) à évaporation de parfum ; dans lequel un conduit venant d'un réservoir à liquide germicide bactéricide (7) se termine dans une zone de sortie d'air chaud, de telle sorte que le liquide germicide bactéricide accède à la zone de sortie ;
dans lequel le réservoir à liquide germicide bactéricide est également situé dans la zone où le purificateur d'air à évaporation est installé, ledit purificateur d'air comportant un dépôt de parfum qui, au moyen d'une mèche (17), sort à l'extérieur à travers une grille supérieure (19) ; (20),
**caractérisé en ce que**
le boîtier (1) comprend une paroi de séparation (2) qui sépare les éléments constituant le sèche-mains d'avec une zone isolée et indépendante ; le dépôt de parfum atteint un élément de chauffage (18) pour faire s'évaporer le parfum ; il y a une grille inférieure d'entrée d'air extérieur ; le liquide germicide bactéricide accède à la zone de sortie au moyen de l'effet Venturi causé par la réduction de pression, une aspiration se faisant au niveau du conduit ; la réduction de pression de l'air chaud cause la pulvérisation du liquide germicide bactéricide vers l'orifice de sortie ; et le conduit de liquide germicide bactéricide comporte une électrovanne (8) dont la fermeture est commandée au moyen d'un circuit électronique (9) commandant les éléments constituant le séchoir.

2. Sèche-mains selon la revendication 1, **caractérisé en ce que** le conduit de sortie pour le liquide germicide bactéricide dans le réservoir a accès à la zone de sortie de séchoir à travers une zone étroite dotée d'une entrée d'air sous pression, l'air sortant à l'extérieur par des trous ou par une buse inférieure, afin d'obtenir la pulvérisation du liquide germicide bactéricide et sa sortie vers les mains pour exécuter la fonction de nettoyage hygiénique.
